# EUROPEAN PATENT SPECIFICATION

(11) **EP 2 523 659 B1**
(45) Date of publication and mention of the grant of the patent: **16.04.2014**
(21) Application number: 11702710.2
(22) Date of filing: 12.01.2011
(51) Int. Cl.: A61K 9/70, A61M 5/142, A61B 5/00, A61M 5/14, A61M 35/00, C09J 7/02

(54) **SKIN MOUNTED MEDICAL PATCH DEVICE**
HAUTMONTIERTES MEDIZINISCHES PFLASTER
DISPOSITIF DE TIMBRE MEDICAL MONTE SUR LA PEAU

(30) Priority: 14.01.2010 CH 40102010
(43) Date of publication of application: 21.11.2012
(73) Proprietor: Sensile Pat AG, 4614 Haegendorf (CH)
(72) Inventor: SCHNIDRIG, Jürg, CH-4457 Diegten (CH); ROBIN, Franck, CH-5600 Lenzburg (CH); BEYER, Uwe, CH-4600 Olten (CH); BRANDT, Derek, CH-4436 Oberdorf (CH)
(74) Representative: reuteler & cie SA
(86) International application number: PCT/IB2011/050130
(87) International publication number: WO 2011/086505

(56) References cited:
- US-A1- 2008 269 687

## Description

The present invention relates to a medical patch device for mounting on skin for analyte sensing and/or drug delivery.

Patch devices for analyte sensing and drug delivery include patch devices for measuring blood glucose levels, and/or for administrating insulin and other drugs for controlling blood sugar levels in diabetes patients. Patch devices may be fixed to the patient's skin for a few hours to a few days. After use, the patch units are disposed of and if needed replaced with a new device.

A medical patch device with a mounting base that has an adhesive layer is disclosed in US2008/0269687. In this device, the adhesive layer may be weakened by a catalyst, however it is difficult to easily and homogenously access the adhesive layer with the catalytic activator and thus difficult to remove a large surface area adhesive layer from the patient in a homogenous, effective and comfortable manner.

It is an object of this invention to provide a patch device for medical sensing or drug delivery applications, such as insulin administration or glucose sensing in diabetes management, that is easy to mount and remove with little discomfort, but that reliably remains in place during use on the patient's skin.

It is advantageous to provide a patch device that is compact and economical to use.

Objects of this invention have been achieved by providing a skin mounted patch device for medical sensing or drug delivery applications according to claim 1.

Disclosed herein is a medical patch device for drug delivery or analyte sensing, the patch device including a mounting base comprising a supporting base layer and an adhesive layer on a mounting side of the supporting base layer, and a functional unit comprising a power supply and an electronic circuit, the functional unit configured to be fixed to the mounting base, wherein the adhesive layer comprises a light switchable adhesive and the support layer is formed as a light guide configured to diffuse light from one or more electronically activated lights sources, the electronically activated light sources being connected to the electronic circuit of the functional device.

For drug delivery applications, the functional unit may further comprise a drug reservoir.

The lights sources may be actionable by means of a button switch on a housing of the functional unit. The light sources are switched on by the user activating the light activation circuit when the user wishes to remove the patch unit. The electronic circuit is configured to switch the electronic lights on for a predetermined amount of time, for instance between 10 to 40 seconds, that irradiates the adhesive to an extent sufficient to perform the switching of the adhesive to a non-tacky state suitable for removal of the mounting base from the skin with low discomfort. The patch unit may be provided with a signalling system indicating when the pre-determined light radiation time of the adhesive is passed so that the user knows when to remove the patch unit with little discomfort. The signalling system may comprise a light indicator, or a message on a display screen on the functional unit, or generate an acoustic signal such as a beeping sound.

In a variant, the electronically activated light sources may be switched on under manual control by the user for as long as the user presses a light switch on the functional unit. The user can apply a certain removal pulling force on the medical patch device until the adhesiveness of the switchable adhesive is reduced sufficiently to comfortably remove the device. In this variant the timing of the light switch on is thus controlled by the user.

The electronically activated light sources may advantageously be in a form of one or more light emitting diodes (LED's). There is preferably a plurality of light sources in a distributed configuration over the mounting base to ensure good light energy density distribution over the entire adhesive layer on the mounting base, which may cover in most applications between 10 to 40 square centimetres. The support layer of the mounting base may comprise cavities or depressions configured for receiving at least a portion of a light source therein so that the light source irradiates at least partially laterally into the light guide in a direction essentially parallel to the adhesive layer.

The mounting base may be configured as a separate component from the functional unit, configured to be removably mounted to the functional unit enabling the mounting base to be disposed of and replaced separately from the functional unit. The adhesive layer may be covered prior to use by means of a removable (for instance peelable), light barrier layer that is removed just prior to fixing the mounting base to the skin of the patient.

The face of the mounting base intended to be mounted against the functional unit may also be provided with a removable (for instance peelable) light barrier layer that is removed just prior to assembling the functional unit on to the mounting base.

The electronic light source or sources may be permanently mounted to the functional unit, at a mounting face of the functional unit. Alternatively, the light sources may be mounted in the mounting base and connected to electrical contacts provided on the mounting base that are configured to be brought into electrical connection with complementary contacts of the functional unit when the functional unit is assembled to the mounting base.

The functional unit may advantageously be formed of two separable units, a single single use disposable unit, and a re-usable unit, the single-use disposable unit comprising for instance a drug reservoir (depending on the application), and the reusable unit comprising the power supply, electronic circuit and for instance at least a portion of a pump (depending on the application). A needle or other implantable member may form part of the disposable unit or of the mounting base. The mounting base and disposable unit may be formed together as a single component, the reusable unit being removable mounted to the disposable unit which includes the switchable adhesive layer.

Further objects and advantageous aspects of the invention will be apparent from the claims and from the following detailed description of embodiments of the invention with reference to the annexed drawings in which:
Figure 1 a is a view in perspective of a functional unit of a patch device according to an embodiment of this invention;
Figure 1b is a view in perspective of a mounting base of a patch device according to an embodiment of this invention;
Figure 1c is a view in perspective of the functional unit of Figure 1 a about to be mounted on the mounting base of Figure 1 b;
Figure 1d is an exploded perspective view of the patch device according to embodiments of Figures 1 a to 1 c;
Figure 2a is a view in perspective of a patch device according to another embodiment of the invention;
Figure 2b is a view in perspective of the embodiment of Figure 2a where the functional unit is being removed from the mounting base;
Figures 3a to 3c are detailed cross-sectional illustrations of light sources of the functional unit irradiating variants of a support layer of the mounting base;
Figures 4a and 4b are detailed cross-sectional illustrations of variants of light sources in the functional unit or the mounting base;
Figures 5a and 5b are detailed cross-sectional illustrations of variants of light sources in the functional unit or the mounting base;
Figure 6 is an exploded view in perspective of another embodiment of the patch device according to the invention where the disposable unit is integrally formed with the mounting base, separable from the reusable unit.

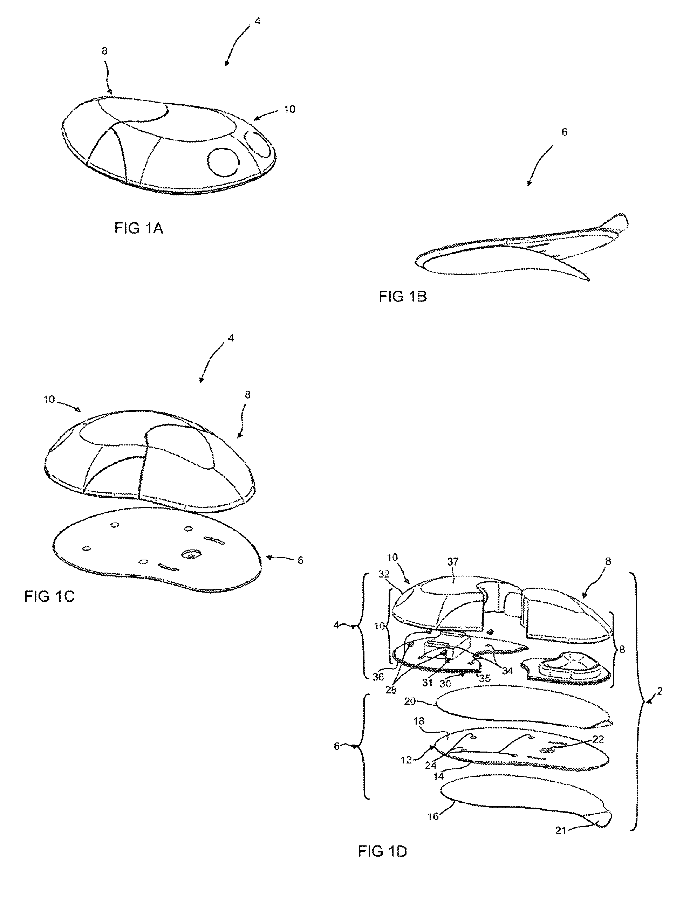

Referring to the figures, especially figures 1a to 1d, a patch device 2 according to an embodiment of the invention comprises a functional unit 4 and a mounting base 6. The functional unit 4 comprises in this embodiment, a single use disposable unit 8 and a reusable unit 10. In a drug delivery application, the disposable unit comprises at least a drug reservoir for containing a drug to be administrated to the patient, and the reusable unit comprising at least a portion of a pump, a power supply, and an electronic circuit. The disposable unit may also comprise a portion of pump which in this case means that the pump is configured in two separable parts, one part in the disposable unit and the other in the reusable unit, the pump being operable when the disposable and reusable units are assembled together. The pump portion in the reusable unit may for example simply comprise a stator portion of the pump for generating an electromagnetic field driving a pump rotor positioned in the disposable unit, or the pump portion in mounted reusable unit could simply comprise a coil or other electromagnetic induction system for supplying power to the pump portion in the disposable unit. The disposable unit may further comprise an implantable member, such as needle or flexible transcutaneous tube for administration of the drug. Alternatively, the implantable member may be provided on the mounting base and comprise an inlet to which the outlet of the reservoir or pump portion in the disposable unit couples to. Advantageously, the reusable unit regroups relatively valuable components that do not need to be disposed of for aging or safety reasons such as the implantable member and consumable drug contained in the disposable section.

The electronic circuit in the reusable unit is configured to control the operation of the pump and administration of drug to the patient. The electronic circuit may have further functions such as to control alarms in the case of malfunction, or to remind the patient for the administration of medication, or to supply readings of analyte levels measured by the patch device. In this regard, the patch device may further comprise an analyte sensing system that senses a body analyte level such as blood sugar by means of the implantable member or a second separate implantable sensing member. The reusable unit may further comprise a display such as an LCD display for providing information on analyte levels or on drug administration doses to the patient, as well as to allow control by the patient of the patch device.

The mounting base 6 comprises a support layer 12 and a light switchable adhesive layer 14 lying against a lower face 17 of the support layer 12, a protective light barrier film 16 covering the adhesive layer 14, the light barrier film 16 being peelable and having a tab portion 21 for better apprehension of the film to be peeled away just prior to sticking the mounting base on a patient's skin.

Light switchable adhesives are adhesives that have strong adhesive properties in their normal state but lose adhesive strength when exposed to visible or ultraviolet light. The adhesive comprises a photo initiator that reacts to light and causing cross-linking of the polymer chains in the adhesive so that it's tackiness reduces. Light switchable adhesives are known *per se* and described in various publications, for instance in US patents 4'268'047, 4'968'559, 6'184'264, and 6'610'762, and thus will not be further described here.

An upper mounting surface 18 of the support layer 12 may be covered with a protective film 20, that may optionally be provided with an adhesive layer to stick onto the support layer 12. The upper adhesive layer may also be provided as a light switchable adhesive layer. In the latter variant, the upper adhesive layer may be used for sticking the functional unit 4 onto the mounting base, both the upper and lower adhesive layers being irradiated by a sufficient amount of light such that the adhesive loses its tackiness and the functional unit can be separated easily from the mounting base and the mounting base separated easily from the patient's skin.

The mounting base 6 and functional unit 4 may alternatively be provided with complementary mechanical fixing means (not shown) such as elastic latches and corresponding latching shoulders to removably clip together the mounting base and functional unit. The mounting base 6 further comprises an orifice 22 configured to allow a needle or other transcutaneous penetrating member of the functional unit to pass therethrough. Alternatively, the orifice 22 may be connectable to an outlet of the pump portion arranged in the disposable unit 8, the orifice 22 extending into an implantable member (not shown) mounted to the support layer 12 and protruding below the lower face thereof for transcutaneous penetration. The upper surface 18 of the support layer 12 may be covered with an opaque coating or other barrier to stop light passing into the support layer from the upper face, except for light passage openings 24 at locations corresponding to the position of light sources. The openings 24 allow light to pass into the support layer for the purpose of irradiating the switchable adhesive layer 14 covering the lower mounting face of the support layer. In a variant, the upper surface 18 of the support layer 12 may also not have a light barrier coating, the passage of light being block by the functional unit 4 when mounted on the mounting base 6.

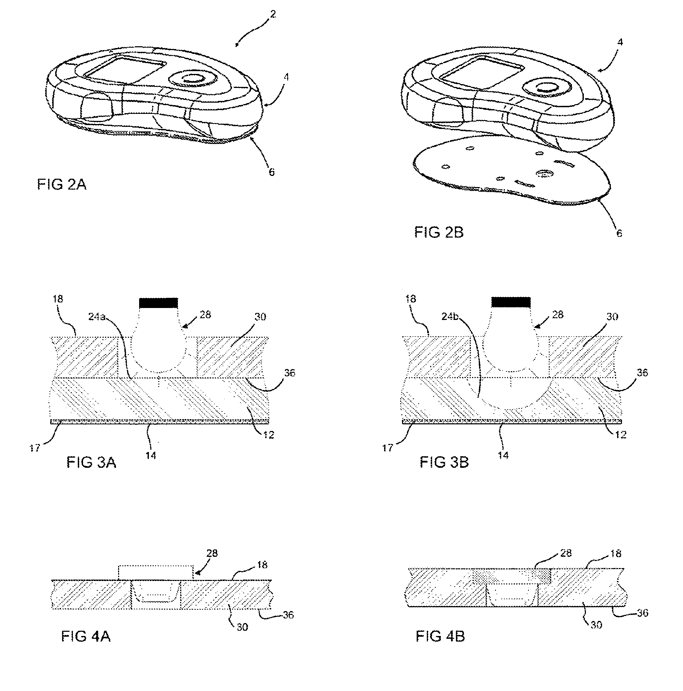

As best illustrated in figures 3a to 3c, the openings 24 may have various shapes, for instance simply a planar surface 24a as in figure 3a, or as shown in the embodiment of figure 3b in the form of a concave lens 24b. The light barrier layer on the upper surface 18 of the support layer may advantageously be in a form of a reflective layer at least on the underside facing the support layer, for example in the form of a thin metal layer formed by vapour deposition or other known thin layer metallization coating techniques. The reflective underside helps to propagate light from a light source over a wide area in the support layer and to avoid losses through the upper layer thus improving irradiation of the adhesive layer on the bottom face of the support layer.

The support layer is made of a light conducting material and acts as a light guide. The support layer may advantageously be made of a light conducting plastics material, such as PMMA, polycarbonate or polystyrene. The support may also advantageously be provided with microstructures, such as micro-prisms or microlenses, as is well known in the prior art, such as in the fabrication of backlighting of large-area panels, for e.g., portable telephones, in order to facilitate the homogeneous diffusion of light. The thickness of the support layer may advantageously lie in a range of 1.5 to 5 millimetres, or preferably from 2 to 3 millimetres in order to ensure a good light distribution over the surface area of the mounting base which may range from 10 to 30 square centimetres.

In order to obtain sufficient irradiation of the light switchable adhesive layer 14 within a user friendly time (for example less than 40 seconds, preferably 10 seconds) there is provided for instance a distribution density of four LEDs for a 20 to 40 cm² area.

The light sources are advantageously light emitting diodes 28 that are mounted on a circuit board 30. In the embodiment shown in figures 1a to 1d, the circuit board 30 is in the functional unit, more specifically in the reusable part 10 of the functional unit, the circuit board also comprising an electronic circuit and circuit components including the power supply 31 and electronic circuit components used for controlling operation of the pump and/or sensor and for controlling the user display and indicators. The electronic light sources 28 may be switched on by the by the user actuating control buttons 32 on the reusable part 10 of the functional unit 4 when the disposable part 8 requires replacement. The light source actuation circuit comprises a timer to ensure that the lights are switched on for a predetermined time configured to switch the switchable adhesive to a non-tacky state.

The light emitting diodes 28 may be mounted in through-cavities 34 of the circuit board 30 such that the light emitting end of the diodes is positioned within the cavity 34, or flush with the lower (mounting) surface 36 of the circuit board. The light emitting diodes could also project beyond the lower mounting surface 36 in which case the support layer 12 of the mounting base is provided with corresponding concave depressions 24c as illustrated in figure 3c.

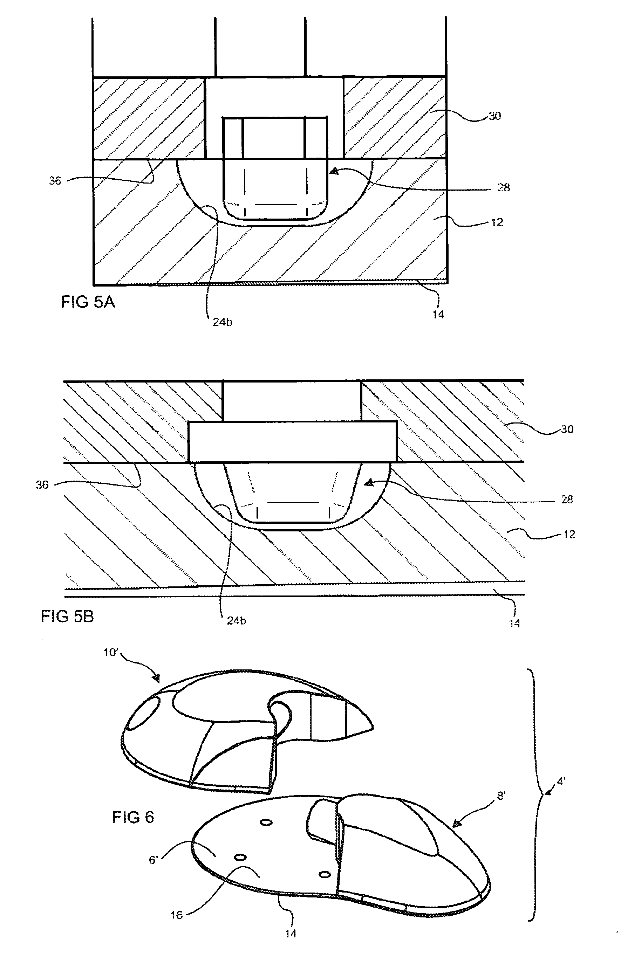

In another variant as illustrated in figure 5a or figure 5b, the light emitting diodes 28 may be mounted on or project below the lower mounting surface 36 of the functional unit 4 so as to be positioned within cavities or depressions 24b in the light guide support layer 12. The protrusion of light emitting diodes into cavities or depressions in the light guide layer 12 has the advantage of increased lateral radiation of light into the support layer for a homogeneous distribution of light over the surface area of the adhesive layer 14.

The circuit board 30 may be mounted on or integrated into a housing base portion 35, for instance made of plastic, that is sealingly assembled to a housing cover portion 37, the housing portions protecting the electronic components of the functional unit from the environment. The housing may comprise lenses or transparent windows to allow the light from the LEDs or other electronically activated light sources to radiate into the support layer 12.

As illustrated in figures 2a and 2b, the functional unit may be formed as a single unit that is mounted to the support layer 12 of the mounting base.

In another variant as illustrated in figure 6, the disposable part 8' of the functional unit 4' may be integrally formed or fixed to the mounting base 6' and provided with a lower light switchable adhesive layer 14 and a protective light barrier film 16 covering the adhesive layer prior to use. In this embodiment there are only two separate parts, the reusable part 10' and the single use disposable part 6'. The advantage of this embodiment is to simplify the mounting of the patch device to the user's skin and the positioning of the reusable unit into the disposable part.

## Claims

1. Medical patch device mountable on skin for drug delivery or analyte sensing, including a mounting base (6) comprising a support layer (12) and an adhesive layer (14) on a mounting side (36) of the support layer, and a functional unit (4) comprising a power supply, and an electronic circuit, the functional unit configured to be fixed to the mounting base, wherein the adhesive layer comprises a light switchable adhesive and the support layer is formed as a light guide configured to diffuse light from one or more electronically activated light sources connected to the electronic circuit of the functional unit.

2. The medical patch device according to claim 1 wherein the electronic circuit is configured to switch the electronically activated light sources on for a predetermined amount of time less than 40 seconds.

3. The medical patch device according to claim 1 or 2 wherein the electronically activated light sources are actionable by a user operated switch provided on a housing of the functional unit.

4. The medical patch device according to claim 1, 2 or 3 wherein the electronically activated light sources are one or more light emitting diodes (LED's).

5. The medical patch device according to any one of the preceding claims wherein there is a plurality of electronically activated light sources in a distributed configuration over the mounting base.

6. The medical patch device according to any one of the preceding claims wherein the support layer of the mounting base comprises cavities or depressions configured for receiving at least portions of said electronically activated light sources therein for radiating light at least partially laterally into the support layer in a direction essentially parallel to the adhesive layer.

7. The medical patch device according to any one of the preceding claims wherein the mounting base is configured as a separate component from the functional unit, and configured to be removably mounted to the functional unit.

8. The medical patch device according to any one of the preceding claims wherein the electronically activated light source or sources are permanently mounted to the functional unit, at a mounting face of the functional unit.

9. The medical patch device according to any one of the preceding claims wherein the functional unit further comprises a drug reservoir.

10. The medical patch device according to claim 9 wherein the functional unit comprises two separable parts, a single-use.disposable part, and a multi-use re-usable part, the single-use disposable part comprising the drug reservoir, and the reusable part comprising the power supply and electronic circuit.

11. The medical patch device according to any one of the preceding claims wherein the mounting base and disposable part are formed together as a single component, configured to removably receive the reusable unit.

12. The medical patch device according to any one of the preceding claims wherein the support layer comprises a light barrier coating on a top side opposite the adhesive layer side.

13. The medical patch device according to claim 12 wherein the light barrier coating is reflective.

14. The medical patch device according to any one of the preceding claims wherein the support layer has one or more lenses facing the configured for distributing light from the one or more electronically activated light sources, the lenses being formed on a top side of the support layer opposite the adhesive layer.

15. The medical patch device according to any one of the preceding claims wherein the support layer has a thickness less than 3 mm and is made from a material of the group consisting of PMMA, polycarbonate or polystyrene.

## Patentansprüche

1. Medizinisches Pflastervorrichtung, auf der Haut montierbar, für die Abgabe von Arzneistoffen oder Analytenabtastung, aufweisend eine Montagebasis (6), die eine Stützschicht (12) und eine Klebstoffschicht (14) auf einer Montageseite (36) der Stützschicht aufweist, und eine Funktionseinheit (4), die eine Stromversorgung und einen elektronischen Schaltkreis aufweist, wobei die Funktionseinheit für ein Befestigen auf der Montagebasis konfiguriert ist, wobei die Klebstoffschicht einen mit Licht schaltbaren Klebstoff aufweist und die Stützschicht als ein Lichtleiter, der für ein Ausbreiten von Licht von einer oder mehreren mit dem elektronischen Schaltkreis der Funktionseinheit verbundenen, elektronisch aktivierten Lichtquellen konfiguriert ist.

2. Medizinisches Pflastervorrichtung nach Anspruch 1, wobei der elektronische Schaltkreis für ein Einschalten der elektronisch aktivierten Lichtquellen für eine im Voraus festgelegte Zeitdauer von weniger als 40 Sekunden konfiguriert ist.

3. Medizinisches Pflastervorrichtung nach Anspruch 1 oder 2, wobei die elektronisch aktivierten Lichtquellen durch einen vom Anwender betätigten, auf einem Gehäuse der Funktionseinheit bereitgestellten Schalter aktivierbar sind.

4. Medizinisches Pflastervorrichtung nach Anspruch 1, 2 oder 3, wobei die elektronisch aktivierten Lichtquellen eine oder mehrere Licht emittierende Dioden (LED) sind.

5. Medizinisches Pflastervorrichtung nach einem der vorhergehenden Ansprüche, wobei mehre elektronisch aktivierte Lichtquellen in einer über die Montagebasis verteilten Konfiguration vorhanden sind.

6. Medizinisches Pflastervorrichtung nach einem der vorhergehenden Ansprüche, wobei die Stützschicht der Montagebasis Hohlräume oder Vertiefungen aufweist, die für die Aufnahme darin von wenigstens Teile der elektronisch aktivierten Lichtquellen für ein wenigstens teilweises seitliches Strahlen von Licht in die Stützschicht in einer im Wesentlich parallelen Richtung zur Klebstoffschicht konfiguriert sind.

7. Medizinisches Pflastervorrichtung nach einem der vorhergehenden Ansprüche, wobei die Montagebasis als eine von der Funktionseinheit separate Komponente konfiguriert und für eine abnehmbare Montage auf der Funktionseinheit konfiguriert ist.

8. Medizinisches Pflastervorrichtung nach einem der vorhergehenden Ansprüche, wobei die elektronisch aktivierte Lichtquelle oder die elektronisch aktivierten Lichtquellen dauerhaft auf einer Montagefläche der Funktionseinheit an der Funktionseinheit angebaut sind.

9. Medizinisches Pflastervorrichtung nach einem der vorhergehenden Ansprüche, wobei die Funktionseinheit ferner einen Arzneistoff-Behälter aufweist.

10. Medizinisches Pflastervorrichtung nach Anspruch 9, wobei die Funktionseinheit zwei separate Teile aufweist, ein Einwegteil für einmalige Anwendung und ein für mehrfache Anwendung wiederverwendbares Teil, wobei das Einwegteil für einmalige Anwendung den Arzneistoff-Behälter aufweist, und das wiederverwendbare Teil die Stromversorgung den elektronischen Schaltkreis aufweist.

11. Medizinisches Pflastervorrichtung nach einem der vorhergehenden Ansprüche, wobei die Montagebasis und das Wegwerfteil zusammen als eine einzelne Komponente geformt sind, die eine abnehmbare Aufnahme des wiederverwendbaren Teils konfiguriert ist.

12. Medizinisches Pflastervorrichtung nach einem der vorhergehenden Ansprüche, wobei die Stützschicht eine Lichtabdeckschicht auf einer Oberseite entgegengesetzt der Klebstoffseite aufweist.

13. Medizinisches Pflastervorrichtung nach Anspruch 12, wobei die Lichtabdeckschicht reflektierend ist.

14. Medizinisches Pflastervorrichtung nach einem der vorhergehenden Ansprüche, wobei die Stützschicht eine oder mehrere Linsen-Außenschichten aufweist, die für ein Verteilen von Licht von der einen oder den mehreren elektronisch aktivierten Lichtquellen konfiguriert sind, wobei die Linsen auf einer Oberseite der Stützschicht entgegengesetzt der Klebstoffschicht geformt sind.

15. Medizinisches Pflastervorrichtung nach einem der vorhergehenden Ansprüche, wobei die Stützschicht eine Dicke von weniger als 3 mm aufweist und aus einem Material ausgewählt aus der Gruppe bestehend aus PMMA, Polycarbonat oder Polystyrol hergestellt ist.

## Revendications

1. Dispositif de patch médical pouvant être placé sur la peau pour l'administration d'un médicament ou la détection d'une substance à analyser, comprenant une base de montage (6) comprenant une couche de support (12) et une couche adhésive (14) d'un côté de montage (36) de la couche de support, et une unité fonctionnelle (4) comprenant une alimentation, et un circuit électronique, l'unité fonctionnelle étant configurée pour être fixée à la base de montage, dans lequel la couche adhésive comprend un adhésif pouvant être commuté par la lumière et la couche de support est formée en tant que guide de lumière configuré pour diffuser la lumière provenant d'une ou de plusieurs sources de lumière activées électroniquement connectées au circuit électronique de l'unité fonctionnelle.

2. Dispositif de patch médical selon la revendication 1, dans lequel le circuit électronique est configuré pour commuter les sources de lumière activées électroniquement pendant une quantité de temps prédéterminée inférieure à 40 secondes.

3. Dispositif de patch médical selon la revendication 1 ou 2, dans lequel les sources de lumière activées électroniquement peuvent être actionnées par un commutateur actionné par un utilisateur prévu sur un boîtier de l'unité fonctionnelle.

4. Dispositif de patch médical selon la revendication 1, 2 ou 3, dans lequel les sources de lumière activées électroniquement sont une ou plusieurs diodes électroluminescentes (DEL).

5. Dispositif de patch médical selon l'une quelconque des revendications précédentes, dans lequel il existe une pluralité de sources de lumière activées électroniquement en une configuration répartie sur la base de montage.

6. Dispositif de patch médical selon l'une quelconque des revendications précédentes, dans lequel la couche de support de la base de montage comprend des cavités ou des renfoncements configurés pour recevoir au moins des parties desdites sources de lumière activées électroniquement dans celle-ci pour rayonner une lumière au moins partiellement latéralement dans la couche de support dans une direction essentiellement parallèle à la couche adhésive.

7. Dispositif de patch médical selon l'une quelconque des revendications précédentes, dans lequel la base de montage est configurée en tant que composant séparé de l'unité fonctionnelle, et est configurée pour être montée de manière amovible sur l'unité fonctionnelle.

8. Dispositif de patch médical selon l'une quelconque des revendications précédentes, dans lequel la source ou les sources de lumière activées électroniquement sont montées de manière permanente sur l'unité fonctionnelle, au niveau d'une face de montage de l'unité fonctionnelle.

9. Dispositif de patch médical selon l'une quelconque des revendications précédentes, dans lequel l'unité fonctionnelle comprend en outre un réservoir de médicament.

10. Dispositif de patch médical selon la revendication 9, dans lequel l'unité fonctionnelle comprend deux parties séparables, une partie jetable à usage unique, et une partie réutilisable à usage multiple, la partie jetable à usage unique comprenant le réservoir de médicament, et la partie réutilisable comprenant l'alimentation et le circuit électronique.

11. Dispositif de patch médical selon l'une quelconque des revendications précédentes, dans lequel la base de montage et la partie jetable sont formées l'une avec l'autre en tant que composant unique, configuré pour recevoir de manière amovible l'unité réutilisable.

12. Dispositif de patch médical selon l'une quelconque des revendications précédentes, dans lequel la couche de support comprend un revêtement formant barrière à la lumière d'un côté supérieur opposé au côté de la couche adhésive.

13. Dispositif de patch médical selon la revendication 12, dans lequel le revêtement formant barrière à la lumière est réfléchissant.

14. Dispositif de patch médical selon l'une quelconque des revendications précédentes, dans lequel la couche de support comporte une ou plusieurs lentilles configurées pour distribuer la lumière provenant desdites une ou plusieurs sources de lumière activées électroniquement, les lentilles étant formées d'un côté supérieur de la couche de support opposé à la couche adhésive.

15. Dispositif de patch médical selon l'une quelconque des revendications précédentes, dans lequel la couche de support a une épaisseur inférieure à 3 mm et est réalisée à partir d'un matériau du groupe consistant en le PMMA, le polycarbonate ou le polystyrène.
